Europäisches Patentamt

European Patent Office

Office européen des brevets

(11) Veröffentlichungsnummer: **0 232 752**
**A1**

## (12) EUROPÄISCHE PATENTANMELDUNG

(21) Anmeldenummer: **87100673.0**

(22) Anmeldetag: **20.01.87**

(51) Int. Cl.⁴: **C07D 301/12** , C07D 301/32 , C07D 303/16

(30) Priorität: **25.01.86 DE 3602254**

(43) Veröffentlichungstag der Anmeldung:
**19.08.87 Patentblatt 87/34**

(84) Benannte Vertragsstaaten:
**BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Hickmann, Eckhard Dr.**
**Kantstrasse 23**
**D-6701 Dannstadt-Schauernheim(DE)**

(54) **Verfahren zur Herstellung von Oxiranylcarbonsäureestern.**

(57) Herstellung von Oxiranylcarbonsäureestern aus ungesättigten Carbonsäureestern durch Epoxidation mit Wasserstoffperoxid in einem wäßrig-organischen Zweiphasensystem in Gegenwart eines Katalysators aus einem Alkaliwolframat, Wolframsäure oder Wolfram(VI)oxid, Phosphorsäure und einem quartären Ammonium-oder Phosphoniumsalz, indem man die Reaktion in Gegenwart von 1 bis 2 mol Wasserstoffperoxid je Mol Carbonsäureester und bei einem pH-Wert der wäßrigen Phase zwischen etwa 2 und 4 durchführt.

EP 0 232 752 A1

## Verfahren zur Herstellung von Oxiranylcarbonsäureestern

Die Erfindung betrifft ein verbessertes Verfahren zur Herstellung von Oxiranylcarbonsäureestern aus ungesättigten Carbonsäureestern durch Epoxidation mit Wasserstoffperoxid in einem wäßrig-organischen Zweiphasensystem in Gegenwart eines Katalysators aus einem Alkaliwolframat, Wolframsäure oder Wolfram(VI)oxid, Phosphorsäure und einem quartären Ammonium-oder Phosphoniumsalz.

In der EP-OS 0 154 490 wird die Herstellung von Alkyl-3,4-epoxybutanoat durch Umsetzung des entsprechenden ungesättigten Esters mit 1,2 mol $H_2O_2$ je Mol Ester im Zweiphasensystem Wasser-1,2-Dichlorethan in Gegenwart von Trimethylcaprylammoniumchlorid und Natriumwolframat sowie Phosphorsäure beschrieben. Der pH-Wert der wäßrigen Phase wird durch Zugabe von Schwefelsäure auf 1,6 eingestellt. Über Selektivitäten und Ausbeuten der nach dieser Methode erhaltenen Oxirane sind keine Angaben gemacht worden.

Nach Venturello et al, DE-OS 30 27 349 und J. Org. Chem. 48, 3831-3833 (1983) lassen sich Olefine mit verdünntem wäßrigem Wasserstoffperoxid in Gegenwart eines Phasentransferkatalysators, entstehend aus einem quartären Ammonium-oder Phosphoniumsalz, Wolframat-oder Molybdationen und Phosphat-oder Arsenationen in Gegenwart einer Mineralsäure epoxidieren.

Um hohe Selektivitäten bei der Epoxidierung zu erzielen, wird ein beträchtlicher Olefinüberschuß über das Wasserstoffperoxid empfohlen. Bereits stöchiometrische Mengen an Olefin führen zu einem deutlichen Selektivitätsabfall um 30 -80 % (siehe J. Org. Chem. 48, Seite 3832). Bedingt durch den hohen Olefinüberschuß ist die für die Wirtschaftlichkeit des Verfahrens entscheidende Oxiranausbeute trotz hoher Selektivität (bezogen auf die in Unterschuß verwendete Komponente $H_2O_2$) mit maximal 51,8 % nur mäßig. Bei einer technischen Produktion von Epoxiden nach Venturello et al. wäre man daher zu einer aufwendigen und in der Regel mit erheblichen Verlusten der empfindlichen Oxirane verbundenen Abtrennung vom nicht umgesetzten Olefin und dessen Rückführung in die Epoxidationsreaktion genötigt.

Über die Isolierung der erhaltenen Oxirane aus dem rohen Reaktonsgemisch werden keine Angaben gemacht. Insbesondere bei Oxiranylessigsäureestern ist die Reindarstellung schwierig, da sie basenkatalysiert sehr leicht zu den isomeren -Hydroxicrotonenestern umlagern (s. Ann. Chim. 1973, 8, 359) und bei der destillativen Aufarbeitung der Reaktionsrohprodukte Basen (tert. Amine) durch die bekannte Thermolyse des zur Katalysator formulierung eingesetzten quartären Ammoniumsalzes entstehen (vgl. z.B. E.V. Dehmlow, S. S. Dehmlow, "Phase Transfer Catalysis", 2. Aufl. 1983, S. 60).

Es bestand daher die Aufgabe, ein technisch geeignetes Verfahren für eine hochselektive mit hohen Olefin-Umsätzen verlaufende Epoxidation von ungesättigten Carbonsäureestern zu finden, so daß sich eine aufwendige Rückführung nicht umgesetzter Olefine erübrigt. Weiterhin sollte aufgabengemäß eine verlustarme Reindarstellung der erhaltenen Oxiranylcarbonsäureester ermöglicht werden.

Demgemäß wurde ein vorteilhaftes Verfahren zur Herstellung von Oxiranylcarbonsäureestern aus ungesättigten Carbonsäureestern durch Epoxidation mit Wasserstoffperoxid in einem wäßrig-organischen Zweiphasensystem in Gegenwart eines Katalysators aus einem Alkaliwolframat, Wolframsäure oder Wolfram(VI)oxid, Phosphorsäure und einem quartären Ammonium-oder Phosphoniumsalz gefunden, welches dadurch gekennzeichnet ist, daß man die Reaktion in Gegenwart von 1 bis 2 mol Wasserstoffperoxid je Mol Carbonsäureester und bei einem pH-Wert der wäßrigen Phase zwischen etwa 2 und 4 durchführt.

Überraschenderweise verläuft die Zweiphasen-Epoxidation von ungesättigten Carbonsäureestern zu Oxiranylcarbonsäureestern mit dem bekannten Katalysatorsystem aus einem Alkaliwolframat, Wolframsäure oder Wolfram(VI)oxid, Phosphorsäure, die z.B. aus einem Phosphat und Mineralsäure auch in situ hergestellt werden kann, und einem quartären Ammonium-oder Phosphoniumsalz auch in Gegenwart von überschüssigem Wasserstoffperoxid bei nahezu quantitativen Umsätzen des Olefins mit hohen Selektivitäten (72 -85 %, bezogen auf $H_2O_2$, 80 -90 %, bezogen auf Olefin). Diese Selektivitäten sind gleich oder sogar größer als die bei partiellem Umsatz der ungesättigten Carbonsäureester (<60 %) mit im Unterschuß eingesetztem Wasserstoffperoxid erzielten Selektivitäten.

Nach dem erfindungsgemäßen Verfahren werden 1 bis 2 mol, insbesondere 1,2 bis 1,5, vorzugsweise 1,1 bis 1,3 mol Wasserstoffperoxid je Mol Carbonsäureester verwendet. Das Wasserstoffperoxid kann zu Anfang der Reaktion mit den übrigen Reaktanten zusammen vorgelegt werden oder während der Reaktion portionsweise oder kontinuierlich zudosiert werden; das gleiche gilt für die übrigen Reaktanten. In einer bevorzugten Ausführungsform legt man einen Teil des Wasserstoffperoxids (ca. 20 mol-%, bezogen auf ungesättigten Carbonsäureester) zusammen mit den übrigen Reaktanten vor und dosiert den Rest im Verlauf einiger Stunden zu. Bevorzugt ist die Verwendung von 30 gew.-%igem wäßrigem Wasserstoffperoxid.

Neben der $H_2O_2$-Menge ist das Einhalten eines pH-Bereiches von ca. 2 bis 4 in der wäßrigen Phase wesentlich. Vorzugsweise liegt der pH-Wert bei 2 bis 3,5. Ein pH-Wert in diesem Bereich stellt sich ein, wenn man die Reaktion mit der bevorzugten Katalysatorkombination, bestehend aus einem löslichen Alkaliwolframat, z.B. $Na_2WO_4$ . 2 $H_2O$, Wolframsäure oder Wolfram(VI)oxid, Phosphorsäure oder einem löslichen Phosphat und der zur Überführung in Phosphorsäure äquivalenten Menge einer Mineralsäure, wie Schwefelsäure, und einem für phasentransferkatalysierte Reaktionen üblichen quartären Ammonium-oder Phosphoniumsalz wie Triethylbenzylammoniumchlorid, Tetrabutylammoniumchlorid, -bromid oder -hydrogensulfat, Dimethyldicetylammoniumchlorid, Tributylhexadecylphosphoniumbromid oder Gemischen verschiedener Ammonium-oder Phosphoniumsalzen durchführt und die Acidität weder durch Zugabe von Mineralsäure noch durch Basenzusatz beeinflußt. Besonders geeignet ist Methyltrioctylammoniumchlorid (Aliquat 336®).

Man kann die Reaktion ohne zusätzliches organisches Lösungsmittel durchführen, bevorzugt ist jedoch die Verwendung eines wasserunlöslichen organischen Lösungsmittels wie eines chlorierten Kohlenwasserstoffs, z.B. Chloroform, Chlorbenzol und insbesondere 1,2-Dichlorethan, eines aromatischen Kohlenwasserstoffs, z.B. Benzol, Xylol und insbesondere Toluol, oder eines in Wasser wenig löslichen Ethers, z.B. Dibutylether und insbesondere Methyl-tert.-butylether.

Die Epoxidierung wird vorzugsweise bei Normaldruck im Temperaturbereich von ca. 5c bis ca. 70°C durchgeführt. Unterhalb und oberhalb dieser Temperaturen erfolgt die Umsetzung zwar auch, jedoch ·stören der verlangsamte Reaktionsverlauf bei Temperaturen unter 50°C bzw. die abfallende Selektivität bei Temperaturen über 70°C.

Der Zeitbedarf für die erfindungsgemäße Durchführung der Epoxidation beträgt in der Regel ca. 3 bis ca. 40 Stunden. Die nach Venturello et al notwendigen kurzen Reaktionszeiten sind für Reaktionen im technischen Maßstab nicht einzuhalten, da einerseits die Dosierung länger dauert und zum anderen die beträchtliche Reaktionswärme in so kurzer Zeit nicht leicht abgeführt werden kann. Daß trotzdem hohe Selektivitäten und Ausbeuten erzielt werden, überrascht, da sich lange Reaktionszeiten ungünstig auf die Selektivitäten auswirken sollten.

Nach beendeter Reaktion können beide Phasen peroxidische Verbindungen enthalten, die in bekannter Weise zerstört werden können, z.B. reduktiv durch Zugabe von beispielsweise Natriumhydrogensulfitlösung oder kataly tisch z.B. durch Zugabe eines Platin-oder Palladiumkatalysators. Anschließend wird das Reaktionsgemisch oder die organische Phase allein vorteilhaft mit einer wäßrigen Base neutralisiert, z.B. mit Natronlauge, Kalkmilch, Natriumcarbonat-oder Kaliumhydrogencarbonatlösung und die organische Phase gegebenenfalls mit Wasser gewaschen.

Beim Versuch, aus vorgereinigten, meist flüssigen Roh-Oxiranylcarbonsäureestern die reinen Produkte durch fraktionierte Destillation zu gewinnen, tritt in aller Regel Zersetzung der empfindlichen Oxirane ein. Die Zersetzung läßt sich überraschenderweise vermeiden, wenn man vor der Destillation eine Dünnschichtverdampfung durchführt. Als Dünnschichtverdampfer kommen die üblicherweise verwendeten Apparate in Frage, z.B. Sambay-Verdampfer. Bevorzugt kann die Dünnschichtverdampfung bei Drucken bis zu 20 mbar und bei Temperaturen unterhalb von 120°C durchgeführt werden. In einer besonders bevorzugten Ausführungsform kann diese Destillation unter Strippen mit Inertgas, z.B. Stickstoff, stattfinden.

### Beispiel 1

Oxiranylessigsäureisobutylester aus Vinylessigsäureisobutylester (diskontinuierliche Fahrweise)

In 400 ml entsalztem Wasser wurden 24,8 g Natriumwolframat-Dihydrat und 17,4 g 85 gew.%ige Phosphorsäure gelöst und 190 ml Toluol sowie 12,0 g Methyltrioctylammoniumchlorid - (Aliquat 336]) hinzugefügt und das Gemisch 15 min. bei Raumtemperatur gerührt. Der pH-Wert der wäßrigen Phase lag bei 2.

Nach Zugabe von 220 g 99,3 gew.%igem Vinylessigsäureisobutylester und 200 g 30 gew.%igem Wasserstoffperoxid ( $\widehat{=}$ 114,8 Mol-%, bezogen auf Olefin) wurde das Reaktionsgemisch unter intensivem Rühren 5 1/2 h auf 69 -71°C erwärmt. Nach Phasentrennung wurden 408,3 g organische Phase mit einem Gehalt von 48,2 Gew.% Oxiranylessigsäureisobutylester und 0,7 Gew.% Vinylessigsäureisobutylester und 598,4 g wäßrige Phase - (pH-Wert: 2,9) mit einem Gehalt von 0,21 Gew.% an Wasserstoffperoxid erhalten. Umsatz:
98,7 % Vinylessigsäureisobutylester
97,9 % an Wasswerstoffperoxid
Selektivität:
82,5 % bezogen auf Vinylessigsäureisobutylester
72.2 % bezogen auf Wassestoffperoxid
Rohausbeute an Oxiranylessig-säureisobutylester
81,1 % bezogen auf eingesetzten Vinylessigsäureisobutylester
Nach gängiger Aufarbeitung des Reaktionsgemisches wurde vor der fraktionierten Destillation über einen Sambay-Verdampfer bei einem Druck von 5 mbar und einer Temperatur von 90°C destilliert.

Ausbeute: 194,8 g 99,3 gew.%iger Oxiranylessigsäureisobutylester $\widehat{=}$ 79,6 % d. Th.

Das NMR-Spektrum des Produktes weist folgende Banden auf (3000 MHZ, Lösungsmittel: CDCl₃, innerer Standard: TMS): δ0,9, 6H (Dublett; δ 1,85 -2,05,, 1H (Septett); δ 2,45 -2,7, (201)H (Multipletts); δ 2,75 -2,9, 1H (Multiplett); 3,2 -3,4, 1H (Multiplett), δ 3,9, 2H - (Dublett).

Vergleichsbeispiel 1

In 300 ml entsalztem Wasser wurden 12,4 g Natriumwolframat-Dihydrat und 8,7 g 85 gew.%ige Phosphorsäure gelöst und 140 ml 1,2-Dichlorethan, 6,0 g Methyltrioctylammoniumchlorid (Aliquat 336]-), 222 g 99,5 gew.%iger Vinylessigsäureisobutylester und 108,4 g 30 gew.%igen Wasserstoffperoxid ( $\widehat{=}$ 61,5 Mol-%, bezogen auf Olefin) hinzugefügt, mit 25 gew.%iger Schwefelsäure einen pH-Wert von 1,6 eingestellt und das Reaktionsgemisch unter intensivem Rühren 1 h auf 69 -70°C erwärmt. Nach Phasentrennung wurden 400,3 g organische Phase mit einem Gehalt von 25,9· Gew.% an Oxiranylessigsäureisobutylester und von 26,6 Gew.% an Vinylessigsäureisobutylester und 435,1 g wäßrige Phase (pH-Wert: 1,7) mit einem Gehalt von 0,69 Gew.% an Wasserstoffperoxid erhalten. Umsatz:
51,8 % an Vinylessigsäureisobutylester
90,8 % an Wasserstoffperoxid
Selektivität:
81,5 % bezogen auf Vinylessigsäureisobutylester
75,6 % bezogen auf Wasserstoffperoxid
Rohausbeute an Oxiranylessigsäureisobutylester:
42,2 % bezogen auf eingesetzten Vinylessigsäureisobutylester

Beispiel 2

Oxiranylessigsäureisobutylester aus Vinylessigsäureisobutylester (halbkontinuierliche Fahrweise)

In 3 000 ml entsalztem Wasser wurden 744 g Natriumwolframat-Dihydrat und 444 g 85 gew.%ige Phosphorsäure gelöst und 5 700 ml Toluol sowie 360 g Methyltrioctylammoniumchlorid (Aliquat 336]-) hinzugefügt und das Gemisch 15 min. bei Raumtemperatur gerührt. Der pH-Wert der wäßrigen Phase lag bei 2,1.

Nach Zugabe von 6 600 g 99,5 gew.%igem Vinylessigsäureisobutylester wurde das Reaktionsgemisch auf 50°C erwärmt und insgesamt 6 790 g 30 gew.%iges Wasserstoffperoxid - ( $\widehat{=}$ 129,6 Mol-%, bezogen auf Olefin) hinzugegeben, wobei die ersten 15 Gew.% innerhalb von 20 min und das restliche Wasserstoffperoxid innerhalb von 3 1/2 h bei 60 -64°C hinzugefügt wurden. Anschließend wurde noch 5 h bei 55 -60°C nachgerührt und dann das Reaktionsgemisch bei Raumtemperatur mit Natriumhydrogensulfitlösung und Natriumcarbonatlösung versetzt. Nach Phasentrennung wurden 12 140 g organische Phase mit einem Gehalt von 52,1 Gew.% an Oxiranylessigsäureisobutylester und 1,6 Gew.% an Vinylessigsäureisobutylester und 19 980 g wäßrige, peroxidfreie Phase erhalten. Umsatz:
97,0 % an Vinylessigsäureisobutylester
84,6 % an Wasserstoffperoxid
Selektivität:
89,2 % bezogen auf Vinylessigsäureisobutylester
75,3 % bezogen auf Wasserstoffperoxid
Rohausbeute an Oxiranylessigsäureisobutylester:
86,6 % bezogen auf eingesetzten Vinylessigsäureisobutylester
Die Isolierung erfolgte wie in Beispiel 1 beschrieben.

Ausbeute: 82,6 % d. Theorie an 98,8 %igem Oxiranylessigsäureisobutylester.

Eine entsprechend Beispiel 2 bei pH 2,9 bis 3,3 und 50°C Reaktionstemperatur durchgeführte Umsetzung, jedoch mit einer Nachrührzeit von 31 anstelle von 5 h führte zu folgendem Ergebnis:
Umsatz:
94,9 % an Vinylessigsäureisobutylester
74,9 % an Wasserstoffperoxid
Selektivität:
87,1 % bezogen auf Vinylessigsäureisobutylester
84,8 % bezogen auf Wasserstoffperoxid
Rohausbeute an Oxiranylessigsäureisobutylester:
82,7 % bezogen auf eingesetzten Vinylessigsäureisobutylester
Ausbeute:
75,8 % d. Theorie an 99,6 %igem Oxiranylessigsäureisobutylester.

Beispiel 3

Analog Beispiel 1 wurden 39,6 g Non-3-encarbonsäure-i-propylester mit 27,2 g 30 %igem Wasserstoffperoxid ( $\widehat{=}$ 120 Mol%) in Gegenwart von 3,22 g Natriumwolframatdihydrat, 2,26 g 85 %iger Phosphorsäure, 13 ml Wasser, 1,56 g Methyltrioctylammoniumchlorid (Aliquat 336],) und 24,7 ml Toluol bei pH 3,1 bis 3,2, 60°C Reaktionstemperatur und 10 h Reaktionsdauer epoxidiert. Nach Sambay-Destillation bei 2 bis 5 mbar und 100°C sowie Niedersiederabtrennung bei 0,5 mbar und 30°C erhielt man 33,9 g eines 95 %igen 3,4-Epoxinonancarbonsäure-i-propylesters $\triangleq$ 75 % d. Th.

Das NMR-Spektrum des Produktes weist folgende Banden auf (Meßbedingungen wie bei Beispiel 1):
$\delta$ 0,9, 3H (Triplett); $\delta$ 1,0 -1,6, 8H (Multiplketts); $\delta$ 1,05, 6H (Dublett); $\delta$ 2,45 -2,65, 2H (Multiplett); $\delta$ 2,75, 1H (Multiplett); $\delta$ 3,05, 1H (Multiplett); $\delta$ 5,05, 1H (Seplett).

Beispiel 4

Analog Beispiel 3 wurden 45,2 g 4,8-Dimethylnon-3-encarbonsäure-n-propylester mit 27,2 g 30 %igem Wasserstoffperoxid ($\triangleq$ 120 Mol%) bei pH 2,5 bis 2,9, 55 bis 60°C Reaktionstemperatur und 5 h Reaktionsdauer epoxidiert. Nach Sambay-Destillation bei 1 bis 4 mbar und 110°C sowie Niedersiederabtrennung bei 0,5 mbar und 30°C erhielt man 32,4 g eines 98 %igen 4,8-Dimethyl-3,4-epoxinonancarbonsäure-n-propylesters $\triangleq$ 70 % d. Th.

Das NMR-Spektrum des Produktes weist folgende Banden auf (Meßbedingungen wie bei Beispiel 1):
$\delta$ 0,9, 6H (Dublett); $\delta$ 0,95, 3H (Triplett); $\delta$ 1,0 -1,8, 12H (Multipletts); $\delta$ 2,45 -2,7, 2H (Multiplett); $\delta$ 3,15, 1H (Pseudoquartett); $\delta$ 4,1, 2H (Triplett).

**Ansprüche**

1. Verfahren zur Herstellung von Oxiranylcarbonsäureestern aus ungesättigten Carbonsäureestern durch Epoxidation mit Wasserstoffperoxid in einem wäßrig-organischen Zweiphasensystem in Gegenwart eines Katalysators aus einem Alkaliwolframat, Wolframsäure oder Wolfram(VI)-oxid, Phosphorsäure und einem quartären Ammonium-oder Phosphoniumsalz, dadurch gekennzeichnet, daß man die Reaktion in Gegenwart von 1 bis 2 mol Wasserstoffperoxid je Mol Carbonsäureester und bei einem pH-Wert der wäßrigen Phase zwischen etwa 2 und 4 durchführt.

2. Verfahren nach Anspruch 1, dadurch gekennzeichnet, daß die Reaktionstemperatur 50 bis 70°C beträgt.

3. Verfahren nach den Ansprüchen 1 und 2, dadurch gekennzeichnet , daß die Reaktionsdauer 3 bis 40 Stunden beträgt.

4. Verfahren nach den Ansprüchen 1 bis 4, dadurch gekennzeichnet, daß Toluol ein Bestandteil der organischen Phase ist.

5. Verfahren nach den Ansprüchen 1 bis 5, dadurch gekennzeichnet, daß als ungesättigter Carbonsäureester Vinylessigsäureester verwendet wird.

6. Verfahren zur Reinigung von nach Anspruch 1 hergestellten Oxiranylcarbonsäureestern, dadurch gekennzeichnet, daß der rohe Oxiranylcarbonsäureester nach Abtrennung der wäßrigen Phase vor der destillativen Reinigung durch eine Dünnschichtverdampfung von höhersiedenden Reaktionsbestandteilen befreit wird.

# EUROPÄISCHER RECHERCHENBERICHT

| | EINSCHLÄGIGE DOKUMENTE | | EP 87100673.0 |

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2/A3 - 0 151 289 (BASF)<br>* Ansprüche 1,4,5 *<br>-- | 1-4 | C 07 D 301/12<br>C 07 D 301/32<br>C 07 D 303/16 |
| A | DE - A1 - 2 916 834 (PRODUITS CHIMIQUES UGINE KUHLMANN)<br>* Ansprüche 1,4,7 *<br>-- | 1-4 | |
| A | EP - A1 - 0 109 273 (MONTEDISON S.P.A.)<br>* Zusammenfassung *<br>-- | 1-4 | |
| D,A | EP - A2/A3 - 0 154 490 (I.S.F. SOCIÈTA PER AZIONI)<br>* Zusammenfassung *<br>---- | 1-4 | |

|  | RECHERCHIERTE SACHGEBIETE (Int. Cl.4) |
|---|---|
|  | C 07 D 301/00<br>C 07 D 303/00 |

Der vorliegende Recherchenbericht wurde für alle Patentansprüche erstellt.

| Recherchenort | Abschlußdatum der Recherche | Prüfer |
|---|---|---|
| ·WIEN | 06-05-1987 | BRUS |